(19) Europäisches Patentamt
European Patent Office
Office européen des brevets

(11) Veröffentlichungsnummer: **0 132 733**
**B1**

(12) # EUROPÄISCHE PATENTSCHRIFT

(45) Veröffentlichungstag der Patentschrift: **11.06.86**

(51) Int. Cl.⁴: **C 07 C 53/50**, C 07 C 51/58, C 07 C 125/065

(21) Anmeldenummer: **84108294.4**

(22) Anmeldetag: **14.07.84**

(54) **Neue Fluorpivalsäurefluoride und Verfahren zu ihrer Herstellung.**

(30) Priorität: **26.07.83 DE 3326875**

(43) Veröffentlichungstag der Anmeldung: **13.02.85 Patentblatt 85/7**

(45) Bekanntmachung des Hinweises auf die Patenterteilung: **11.06.86 Patentblatt 86/24**

(84) Benannte Vertragsstaaten: **BE CH DE FR GB IT LI NL**

(56) Entgegenhaltungen: **EP - A - 0 070 463**

(73) Patentinhaber: **BAYER AG, Konzernverwaltung RP Patentabteilung, D-5090 Leverkusen 1 Bayerwerk (DE)**

(72) Erfinder: **Hagemann, Hermann, Dr.,** *Kandinsky-Strasse 52, D-5090 Leverkusen 1* **(DE)**
Erfinder: **Klauke, Erich, Dr., Eichendorffweg 8, D-5068 Odenthal (DE)**
Erfinder: **Kysela, Ernst, Dr., Virchowstrasse 14, D-5060 Bergisch-Gladbach 3 (DE)**

**EP 0 132 733 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1986

**Beschreibung**

Die Erfindung betrifft neue Fluorpivalsäurefluoride und ein Verfahren zu ihrer Herstellung. Die neuen Fluorpivalsäurefluoride können als Zwischenprodukte in weiten Bereichen der organischen Chemie für Synthesezwecke eingesetzt werden, wobei die Gebiete der Pharmazeutika, Pflanzenschutzmittel, Farbstoffe, Kunststoffe und Kunststoffhilfsprodukte beispielhaft genannt seien. Besonders vorteilhaft können die neuen Verbindungen für die Synthese von Synergisten auf dem Gebiet der Insektizide verwendet werden.

Es wurden nun die neuen Fluorpivalsäurefluoride der allgemeinen Formel (I) gefunden

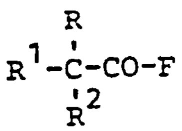

(I)

in welcher

R, $R^1$ und $R^2$ unabhängig voneinander für $CH_3$ oder $CH_2F$ stehen, wobei wenigstens einer der Reste R, $R^1$ und $R^2$ $CH_2F$ bedeutet.

Weiterhin wurde gefunden, daß man die Fluorpivalsäurefluoride der allgemeinen Formel (I)

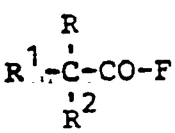

(I)

in welcher

R, $R^1$ und $R^2$ unabhängig voneinander $CH_3$ oder $CH_2F$ bedeuten, wobei wenigstens einer der Reste R, $R^1$ und $R^2$ $CH_2F$ bedeutet,

erhält,

wenn man entsprechende Chlorpivalsäurechloride der allgemeinen Formel (II)

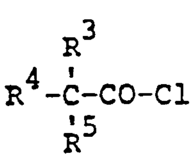

(II)

in welcher $R^3$, $R^4$ und $R^5$ unabhängig voneinander für $CH_3$ oder $CH_2Cl$ stehen, wobei wenigstens einer der Reste $R^3$, $R^4$ und $R^5$ $CH_2Cl$ bedeutet,

mit Metallfluoriden bei erhöhten Temperaturen, gegebenenfalls in Gegenwart eines Lösungsmittels, umsetzt und die Verbindungen der Formel (I) nach üblichen Methoden isoliert und gegebenenfalls reinigt.

Die erfindungsgemäß als Ausgangsstoffe der allgemeinen Formel (II) einzusetzenden Verbindungen sind bekannt und/oder können nach allgemein bekannten Verfahren und Methoden aus den jeweiligen Carbonsäuren hergestellt werden.

Es ist als ausgesprochen überraschend anzusehen, daß im Verlaufe des erfindungsgemäßen Verfahrens der Chlor/Fluor-Austausch bei der als besonders reaktionsträge bekannten Neopentyl-Struktur im Falle der Chlorpivaloylchloride in so glatter Reaktion und guter Ausbeute verläuft, zumal sich z.B. Dichlorpinakolin unter den entsprechenden Bedingungen nur mit maximal 20 % Ausbeute in das entsprechende Difluorpinakolin überführen läßt.

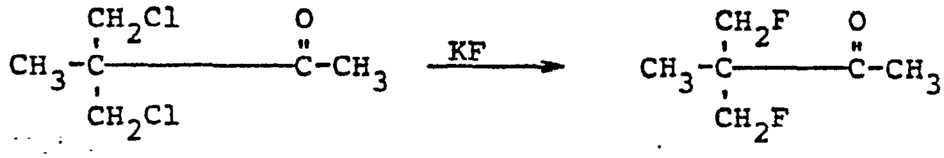


Setzt man beispielsweise Dichlorpivaloylchlorid mit Kaliumfluorid um, so kann das erfindungsgemäße Verfahren durch das folgende Formelschema erläutert werden:

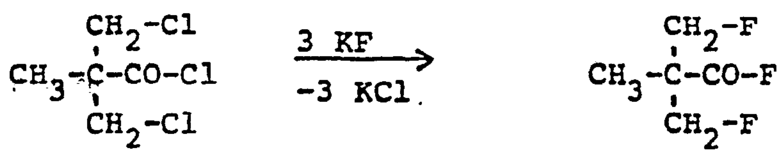


Beim erfindungsgemäßen Verfahren werden als Metallfluoride vorzugsweise die Alkalifluoride, wie Natriumund Kaliumfluorid (besonders bevorzugt Kaliumfluorid) eingesetzt.

Auf 1 Mol der Ausgangsverbindungen der allgemeinen Formel (II) werden wenigstens so viele Mole des Metallfluorids, also z.B. Kaliumfluorid eingesetzt, wie Chloratome durch Fluoratome zu ersetzen sind, wobei das Säurechlorid-Chloratom mit zu berücksichtigen ist. So verwendet man zur Umsetzung des Monochlorpivalsäurechlorids wenigstens 2, des Dichlorpivalsäurechlorids wenigstens 3 und des Trichlorpivalsäurechlorids wenigstens 4 Mol des Metallfluorids, z.B. des Kaliumfluorids. Es kann vorteilhaft sein, das Metallfluorid (z.B. Kaliumfluorid) in einem Überschuß von 5 bis 15, vorzugsweise 8 bis 12 Mol-% je auszutauschendem Chloratom einzusetzen. Die Reaktion kann bei Normaldruck oder auch bei erhöhtem Druck durchgeführt werden. Gegebenenfalls wird die Reaktion unter einem Inertgas wie Stickstoff oder Helium vorgenommen.

Das erfindungsgemäße Verfahren wird bei erhöhten Temperaturen, vorzugsweise zwischen 100 und 300°C und besonders bevorzugt zwischen 130 und 250°C durchgeführt.

Als Lösungsmittel kommen alle für die Reaktion inerten organischen Lösungsmittel in Frage.

Hierzu gehören insbesondere aliphatische und aromatische, gegebenenfalls halogenierte Kohlenwasserstoffe, wie Pentan, Hexan, Heptan, Cyclohexan, petrolether, Benzin, Ligroin, Benzol, Toluol, Xylol, Methylenchlorid, Ethylenchlorid, Chloroform, Tetrachlorkohlenstoff, Chlorbenzol, o-Dichlorbenzol, Ether wie Diethyl- und Dibutylether, Glykoldimethylether und Diglykoldimethylether, Tetrahydrofuran und Dioxan, Ketone, wie Aceton, Methylethyl-, Methylisopropyl- und Methylisobutylketon, Ester, wie Essigsäuremethylester und -ethylester, Nitrile, wie z.B. Acetonitril und Propionitril, Amide, wie z.B. Dimethylformamid, Dimethylacetamid und N-Methylpyrrolidon, sowie Dimethylsulfoxid, Tetramethylensulfon und Hexamethylphosphorsäuretriamid sowie Gemische dieser Lösungsmittel. Als besonders bevorzugt wird Tetramethylensulfon (gegebenenfalls in Mischung mit einem der obengenannten Lösungsmittel) verwendet.

Wie bereits oben erwähnt, können die neuen Verbindungen der allgemeinen Formel (I) für eine Vielzahl von chemischen Synthesen eingesetzt werden.

So kann man die Verbindungen der Formel (I) in bekannter Weise (vgl. z.B. Methoden der organischen Chemie, Houben-Weyl, Band IX/I (1957), Seiten 867-872, Thieme-Verlag, Stuttgart) in die Isocyanate überführen, welche mit Propargylalkohol Carbamidsäureester ergeben, welche in überraschender Weise in Mischungen mit arthropodiziden Wirkstoffen eine starke synergistische Wirkung zeigen und somit als Schädlingsbekämpfungsmittel, insbesondere in Insektiziden, verwendet werden können.

Zur Herstellung der Isocyanate werden die Fluorpivaloylfluoride z.B. in einer Lösung in Aceton mit etwa molaren Mengen Natriumazid in wäßriger Lösung bei etwa 0 bis 20°C umgesetzt. Anschließend wird mit Toluol extrahiert, die Toluolphase abgetrennt, mit Natriumsulfat getrocknet und die Toluol-Lösung zum Sieden erhitzt. Das entstandene Isocyanat kann direkt in der Toluol-Lösung umgesetzt werden oder durch Destillation isoliert werden.

Die Isocyanate werden nach allgemein üblichen Methoden mit dem Propargylalkohol umgesetzt, z.B. in molaren Mengen unter Hinzufügung von katalytischen Mengen Diazabicyclooctan bei Raumtemperatur. Die Herstellung der Carbamidsäureester kann durch das folgende Reaktionsschema verdeutlicht werden:

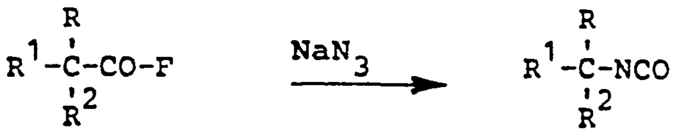


(1) (2)

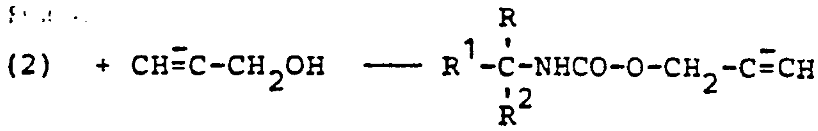

(wobei R, $R^1$ und $R^2$ die oben angegebene Bedeutung haben).

Das erfindungsgemäße Verfahren kann durch die folgenden Herstellungsbeispiele erläutert werden:

**Beispiel 1**

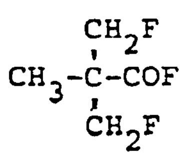

aus

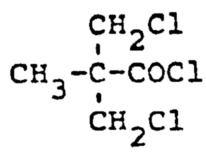

Es werden 947,5 g (5 Mol) Dichlorpivaloylchlorid, 2 kg Tetramethylensulfon und 1,16 kg (20 Mol) Kaliumfluorid bei einem Anfangsdruck von 2 bar $N_2$ für 5 Stunden bei 230°C in einem VA-Rührautoklaven erhitzt, abgekühlt, entspannt und unter leicht vermindertem Druck destilliert. Man erhält 576 g (79,2 % der Theorie) Difluorpivaloylfluorid (Kp. $_{110\ 120\ mbar}$ 52-56°C). Im 3 kg-Maßstab in einem Schaufelreaktor erhält man Ausbeuten zwischen 85 und 90 % der Theorie.

**Beispiel 2**

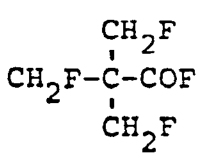

aus

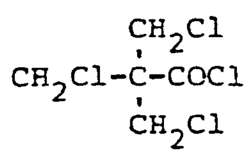

Es werden 900 g (4 Mol) Tris-chlormethylessigsäurechlorid (Trichlorpivaloylchlorid), 1,16 kg (20 Mol) Kaliumfluorid und 2250 ml Tetramethylensulfon unter Normaldruck 5 Stunden bei 200°C gerührt, abgekühlt, mit 1

1 Xylol versetzt und bis zum Siedepunkt des Tetramethylensulfons andestilliert. Die Xylollösung enthält das gesamte Trisfluormethylessigsäurefluorid (80 % der Theorie), das wegen seines Schmelzpunkts von 50-52° C, des Siedepunkts von 48° C/22 mbar und der hohen Reaktivität des Säurefluorids im allgemeinen in der Lösung direkt weiter umgesetzt wird.

**Beispiel 3**

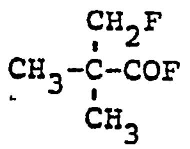

aus

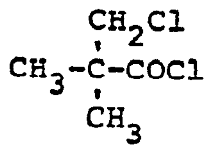

5,8 kg (100 Mol) Kaliumfluorid und 7,7 kg Tetramethylensulfon werden in einem 20 1 Schaufelreaktor mit Destillationsaufbau vorgelegt und bei ca. 20 mbar unter Abnahme von 10 % des eingesetzten Lösungsmittels andestilliert. Es wird mit $N_2$ belüftet, die Innentemperatur von 150° auf 125-130° C fallengelassen, 6,2 kg (40 Mol) Chlorpivaloylchlorid eingesaugt, die Apparatur mit $N_2$ gespült und druckdicht verschlossen. Nach Aufpressen von 3 bar $N_2$ wird 1 Stunde auf 150° C und 12 Stunden auf 230° C erwärmt, auf 80° C abgekühlt und bei 100 mbar destilliert. Man erhält 3,256 kg (68 %) Fluorpivaloylfluorid (Siedepunkt 40-41° C/IOO mbar) und 1,4 kg (26 %) ChlorPivaloylfluorid (Siedepunkt 65° C/100 mbar) als Nebenprodukt. Das Ergebnis entspricht einer Selektivität von 92 % bei 74 % Umsatz.

Die Herstellung der synergistisch wirkenden Carbamidsäureester aus den erfindungsgemäßen Verbindungen der Formel (I) über die Isocyanate und anschließende Umsetzung mit Propargylalkohol kann durch das folgende Beispiel erläutert werden:

**Beispiel 1 A**

N-(1,1-Bis-fluormethyl-ethyl)-carbamidsäure-0-2-propinyl-ester

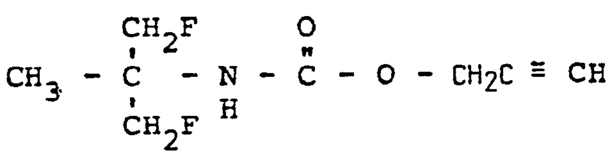

14 g (0,1 Mol) 2,2-Bisfluormethylpropansäurefluorid werden in 200 ml Aceton gelöst. Hierzu tropft man bei 0° C eine Lösung von 6,5 g (0,1 Mol) Natriumazid in 20 ml Wasser und rührt eine Stunde bei Raumtemperatur (ca. 20° C). Die wäßrige Phase wird zweimal mit je 200 ml Toluol extrahiert und die resultierende Toluolphase zweimal mit je 200 ml Wasser gewaschen. Nach Trocknen über Natriumsulfat wird die Toluolphase langsam bis zum Ende der auftretenden Gasentwicklung auf 70-80° C erhitzt. Anschließend kocht man eine Stunde am Rückfluß. Die Vollständigkeit der Bildung des 1,1-Bisfluormethyl-ethylisocyanats wird IR-spektroskopisch verfolgt (Abnahme $\nu_{CON} = 2120$, Zunahme $\nu_{N-C=O} = 2250\ cm^{-1}$). Zu der erhaltenen Lösung tropft man bei ca. 20° C 11,2 g 2-Propinylalkohol und gibt 10 mg Diazabicyclooctan (Dabco) zu.

Nach vierstündigem Erhitzen des Gemisches zum Sieden wird das Reaktionsgemisch abgekühlt und mit Wasser gewaschen. Nach Trocknen über Natriumsulfat wird das Lösungsmittel abdestilliert und das zurückbleibende öl im Hochvakuum vom restlichen Lösungsmittel befreit. Man erhält 13 g N-(1,1-Difluormethylethyl) -carbamidsäure-0-2-propinylester in Form eines farblosen Öls (68 % der Theorieausbeute), $n^{20}$: 1,4430.

Entsprechend können auch die übrigen erfindungsgemäßen Verbindungen der Formel (I) in die Carbamidsäureester-Synergisten übergeführt werden:

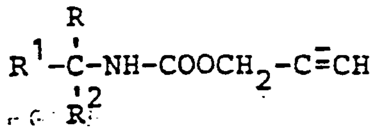

Beispiel R $R^1$ $R^2$ Brechungsindex Nr. $n_D^{20}$ 2A $CH_2F$ $CH_2F$ $CH_2F$ 1,4371 3A $CH_2F$ $CH_3$ $CH_3$ 1,4470

Die überraschende synergistische Wirksamkeit der Carbamidsäureester soll anhand der folgenden Testergebnisse gezeigt werden, wobei der insektizide Wirkstoff Propoxur der Formel

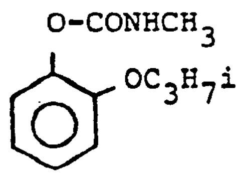

als insektizide Komponente dient. Angewendete Testmethode: $LT_{100}$-Test

Testtiere: Musca domestica ♀♀, Stamm Weymanns (resistent)

Lösungsmittel: Aceton

Von den Wirkstoffen, Synergisten und Gemischen aus Wirkstoffen und Synergisten werden Lösungen hergestellt und 2,5 ml davon in Petrischalen auf Filterpapier von 9,5 cm Durchmesser pipetiert. Das Filterpapier saugt die Lösungen auf. Die Petrischalen bleiben so lange offen stehen, bis das Lösungsmittel vollständig verdunstet ist. Anschließend gibt man 25 Testtiere in die Petrischalen und bedeckt sie mit einem Glasdeckel.

Der Zustand der Tiere wird bis zu 6 Stunden fortlaufend kontrolliert. Es wird diejenige Zeit ermittelt, die für eine 100 %ige knock down-Wirkung erforderlich ist. Wird die $LT_{100}$ nach 6 Stunden nicht erreicht, wird der % Satz der knock down gegangenen Testtiere festgestellt.

Wirkstoffe, Synergisten, die Konzentrationen der Wirkstoffe, Synergisten und Gemische sowie ihre Wirkungen gehen aus der nachfolgenden Tabelle hervor.

Testergebnisse

| Wirkstoff | Synergist (Bsp. Nr) | Konzentration in % Wirkstoff | Synergist | $LT_{100}$ in Min. oder bei 360' in % |
|---|---|---|---|---|
| (A) | — | 1,0 | — | 360' = 60 % |
| — | 1A | — | 0,2 | 360' = 20 % |
| — | 2A | — | 0,2 | 360' = 90 % |
| — | 3A | — | 0,2 | 360' = 20 % |
| (A) | 1A | 0,04 + | 0,04 | 90' |
| (A) | 2A | 0,04 + | 0,04 | 90' |
| (A) | 3A | 0,04 + | 0,04 | 75' |

Die Schädlingsbekämpfungsmittel, welche die Synergisten enthalten, können nach den allgemein üblichen Methoden angewandt werden, z.B. in Form von Sprays von Spritzpulver usw.

**Patentansprüche**

1. Verbindungen der allgemeinen Formel (I)

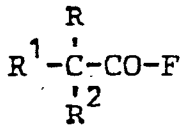

(I)

in welcher

$R, R^1$ und $R^2$ unabhängig voneinander für $CH_3$ oder $CH_2F$ stehen, wobei wenigstens einer der Reste R, R und R $CH_2F$ bedeutet.

2. Verbindung der Formel

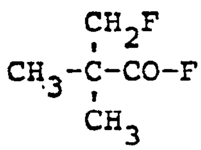

3. Verbindung der Formel

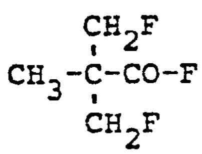

4. Verbindung der Formel

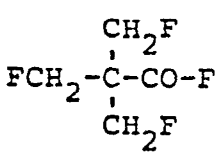

5. Verfahren zur Herstellung von Verbindungen der allgemeinen Formel (I)

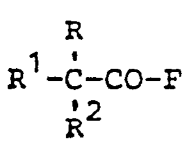

(I)

in welcher

$R, R^1$ und $R^2$ unabhängig voneinander für $CH_3$ oder $CH_2F$ stehen, wobei wenigstens einer der Reste $R, R^1$ und $R^2$ $CH_2F$ bedeutet,

dadurch gekennzeichnet, daß man Verbindungen der allgemeinen Formel (II)

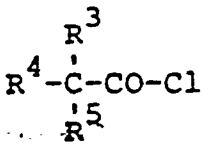

(II)

in welcher

$R^3$, $R^4$ und $R^5$ unabhängig voneinander für $CH_3$ oder $CH_2Cl$ stehen, wobei wenigstens einer der Reste $R^3$, $R^4$ und $R^5$ $CH_2Cl$ bedeutet,

mit Metallfluoriden bei erhöhten Temperaturen, gegebenenfalls in Gegenwart eines Lösungsmittels umsetzt und die Verbindungen der Formel (I) nach üblichen Methoden isoliert und gegebenenfalls reinigt. Verfahren gemäß Anspruch 5, dadurch gekennzeichnet daß man die Umsetzung in Gegenwart des Lösungsmittels Tetramethylensulfon vornimmt.

7. Verfahren gemäß den Ansprüchen 5 und 6, dadurch gekennzeichnet, daß man als Metallfluorid Kaliumfluorid einsetzt.

8. Verfahren gemäß den Ansprüchen 5 bis 7, dadurch gekennzeichnet, daß man die Umsetzung bei Temperaturen zwischen 100 und 300°C, vorzugsweise 130 und 250°C vornimmt.

## Claims

1. Compounds of the general formula (I)

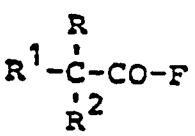

(I)

in which

R, $R^1$ and $R^2$ inecpendently of one another represent $CH_3$ or $CH_2F$, at least one of the radicals R, $R^1$ and $R^2$ denoting $CH_2F$.

2. Compound of the formula

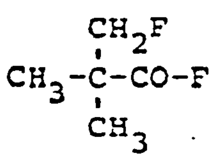

3. Compound of the formula

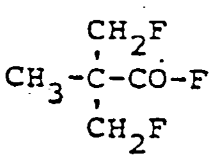

4. Compound of the formula

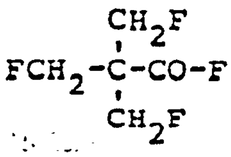

5. Process for the preparation of compounds of the general formula (I)

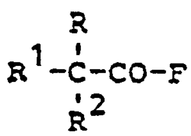 (I)

in which
R, $R^1$ and $R^2$ independently of one another represent $CH_3$ or $CH_2F$, at least one of the radicals R, $R^1$ and $R^2$ denoting. $CH_2F$,
characterised in that compounds of the general formula

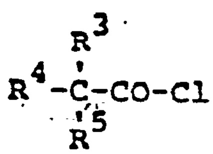 (II)

in which
$R^3$, $R^4$ and $R^5$ independently of one another represent $CH_3$ or $CH_2Cl$, at least one of the radicals $R^3$, $R^4$ and $R^5$ denoting $CH_2Cl$,

are reacted with metal fluorides at elevated temperatures, if appropriate in the presence of a solvent, and the compounds of the formula (I) are isolated, and if appropriate purified, by customary methods.

6. Process according to Claim 5, characterised in that the reaction is carried out in the presence of the solvent tetramethylene sulphone.

7. Process according to Claims 5 and 6, characterised in that potassium fluoride is used as the metal fluoride.

8. Process according to Claims 5 to 7, characterised in that the reaction is carried out at temperatures between 100 and 300°C, preferably 130 and 250°C.

**Revendications**

1. Composés de formule générale (I)

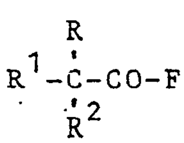 (I)

dans laquelle
R, $R^1$ et R représentent, indépendamment l'un de l'autre, $CH_3$ ou $CH_2F$, l'un au moins des restes R, $R^1$ et $R^2$ représentant $CH_2F$.

2. Composé de formule

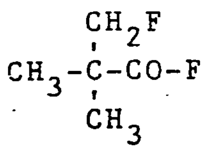

3. Composé de formule

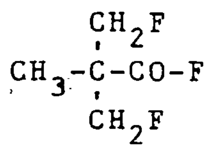

4. Composé de formule

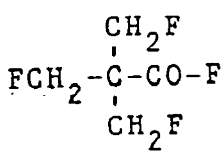

5. Procédé de production de composés de formule générale (I)

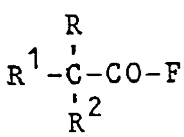

(I)

dans laquelle

R, $R^1$ et $R^2$ représentent, indépendamment l'un de l'autre, $CH_3$ ou $CH_2F$, l'un au moins des restes R, $R^1$ et $R^2$ représentant $CH_2F$,

caractérisé en ce qu'on fait réagir des composés de formule générale (II)

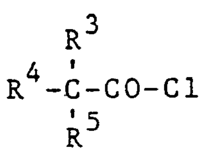

(II)

dans laquelle

$R^3$, $R^4$ et R représentent, indépendamment l'un de l'autre, $CH_3$ ou $CH_2Cl$, l'un au moins des restes $R^3$, $R^4$ et $R^5$ représentant $CH_2Cl$,

avec des fluorures métalliques à des températures élevées, éventuellement en présence d'un solvant et on isole les composés de formule (I) et on les purifie éventuellement par des opérations classiques.

6. Procédé suivant la revendication 5, caractérisé en ce qu'on conduit la réaction en présence de tétraméthylènesulfone utilisée comme solvant.

7. Procédé suivant les revendications 5 et 6, caractérisé en ce qu'on utilise le fluorure de potassium comme fluorure métallique.

8. Procédé suivant les revendications 5 à 7, caractérisé en ce qu'on conduit la réaction à des températures comprises entre 100 et 300°C, de préférence entre 130 et 250°C.